# EUROPEAN PATENT SPECIFICATION

(11) **EP 3 659 642 B1**
(45) Date of publication and mention of the grant of the patent: **29.03.2023**
(21) Application number: 19210460.2
(22) Date of filing: 20.11.2019
(51) Int. Cl.: A61M 1/16

(54) **HEMODIALYSIS MACHINE**
HAEMODIALYSEMASCHINE
APPAREIL D'HÉMODIALYSE

(30) Priority: 30.11.2018 JP 2018225374
(43) Date of publication of application: 03.06.2020
(73) Proprietor: Shibuya Corporation, Kanazawa-shi, Ishikawa 920-8681 (JP)
(72) Inventor: SAWADA, Toshiharu, Kanazawa-shi, Ishikawa 920-8681 (JP); SAITO, Shingo, Kanazawa-shi, Ishikawa 920-8681 (JP); MISHIMA, Takashi, Kanazawa-shi, Ishikawa 920-8681 (JP); NAKAMURA, Yuki, Kanazawa-shi, Ishikawa 920-8681 (JP)
(74) Representative: Mewburn Ellis LLP

(56) References cited:
- EP-A1- 2 878 318
- US-A- 5 895 578
- US-B1- 6 448 062

## Description

### Field of the Invention

The present invention relates to a hemocatharsis apparatus, and specifically to a technique for cleaning a dialysate circuit. The present invention especially relates to a hemocatharsis apparatus having a dialysate circuit provided with a water removal passage and a water removal metering pump disposed in the water removal passage.

### Description of the Related Art

As the generally employed hemocatharsis apparatus for the use in the hemodialysis treatment, Japanese Patent No. 6332605 discloses the hemocatharsis apparatus having the dialysate circuit for distribution of the dialysate, in which the water removal passage and the water removal metering pump are provided for the purpose of removing waste and discharging surplus water from the patient under the hemodialysis treatment.

The above-described hemocatharsis apparatus that has been used for the treatment is required to clean the dialysate circuit after termination of the treatment. For this reason, a closed circuit is formed by the respective passages constituting the dialysate circuit so that the cleaning liquid is circulated through the closed circuit.

In this circumstance, the high-concentration undiluted cleaning liquid supplied to the respective passages is circulated through the closed circuit together with purified water so that the undiluted cleaning liquid is diluted to the predetermined concentration.

As the metering pump used for the hemocatharsis apparatus, the so called piston pump is known as disclosed in Japanese Patent No. 4089530. The ceramic as the material excellent in wear resistance and durability is used for forming the cylinder and the piston that constitute the piston pump.

Assuming that citric acid is used as the cleaning liquid, the use of such cleaning liquid that is kept undiluted with high concentration for cleaning the metering pump provided with the ceramic members may cause corrosion of the ceramic members constituting the metering pump.

In consideration of the above-described problem, it is an object of the present invention to provide the hemocatharsis apparatus capable of preventing damage to the metering pump owing to the high-concentration cleaning liquid.
EP 2878318 A1 discloses a dialysis apparatus adapted to clean a dialysate circuit by circulating a heated cleaning liquid therethrough.
US 5895578 A discloses a method for disinfection of the hydraulic circuit of a dialysis machine.
US 6448062 B1 discloses a composition for simultaneous cleaning and decontamination of medical devices.

### SUMMARY OF THE INVENTION

The hemocatharsis apparatus according to the present invention is as set out in claim 1.

The hemocatharsis apparatus includes a metering container having a supply chamber for containing a fresh dialysate, and a recovery chamber for containing a used dialysate, a dialysate supply passage for supplying the fresh dialysate from the supply chamber to a hemocatharsis device, a dialysate recovery passage for recovering the used dialysate from the hemocatharsis device to the recovery chamber, a dialysate pump disposed in the dialysate recovery passage, a water supply passage for supplying purified water to the supply chamber, a water supply pump disposed in the water supply passage, a draining passage for draining the used dialysate from the recovery chamber, a water removal passage for connecting the dialysate recovery passage and the draining passage while bypassing the metering container, a water removal metering pump disposed in the water removal passage, and a control means for controlling operations of the water removal metering pump, the water supply pump and the dialysate pump. The apparatus is configured such that a closed circuit is formed of a connection passage for connecting a downstream side of the dialysate supply passage and an upstream side of the dialysate recovery passage while bypassing the hemocatharsis device, and a circulation passage for connecting an upstream side of the water supply passage and a downstream side of the draining passage, at the time of cleaning a dialysate circuit constituted by the respective passages. A cleaning liquid is distributed to the closed circuit for cleaning the dialysate circuit. A determination unit is provided for determining whether a concentration of the cleaning liquid in the closed circuit is appropriate. The hemocatharsis apparatus is configured such that in a state in which the water removal metering pump is stopped, the control means is configured to operate the water supply pump such that a predetermined amount of undiluted cleaning liquid is drawn into the closed circuit from a cleaning liquid tank, and is further configured to operate the water supply pump and the dialysate pump to circulate purified water and the undiluted cleaning liquid through the closed circuit, such that the cleaning liquid is prepared by diluting the undiluted cleaning liquid with the purified water in circulation through the closed circuit. When it is determined by the determination unit that the concentration of the cleaning liquid in the closed circuit is appropriate, the water removal metering pump is activated to distribute the cleaning liquid to the water removal passage and the water removal metering pump.

According to the invention, while the high-concentration undiluted cleaning liquid is circulating through the closed circuit formed in the dialysate circuit, the metering pump is stopped so as not to come in contact with the high-concentration undiluted cleaning liquid for preventing damage to members constituting the metering pump.

Upon determination of the determination unit that the concentration of the cleaning liquid in the closed circuit is appropriate, the metering pump is activated, and cleaned using the cleaning liquid with appropriate concentration.

### BRIEF DESCRIPTION OF THE DRAWINGS

FIG. 1 illustrates a structure of a hemocatharsis apparatus according to an embodiment;
FIG. 2 is a sectional view of a metering pump;
FIG. 3 is an explanatory view of an operation in cleaning for drawing the undiluted cleaning liquid to a dialysate circuit;
FIG. 4 is an explanatory view of an operation in cleaning for circulating the cleaning liquid through a first closed circuit;
FIG. 5 is an explanatory view of an operation in cleaning for circulating the cleaning liquid through the first closed circuit;
FIG. 6 is an explanatory view of an operation in cleaning for circulating the cleaning liquid through the first closed circuit;
FIG. 7 is an explanatory view of an operation in cleaning for circulating the cleaning liquid through a second closed circuit; and
FIG. 8 is an explanatory view of an operation for drawing the undiluted cleaning liquid into the dialysate circuit according to a second embodiment.

### DETAILED DESCRIPTION OF THE PREFERRED EMBODIMENTS

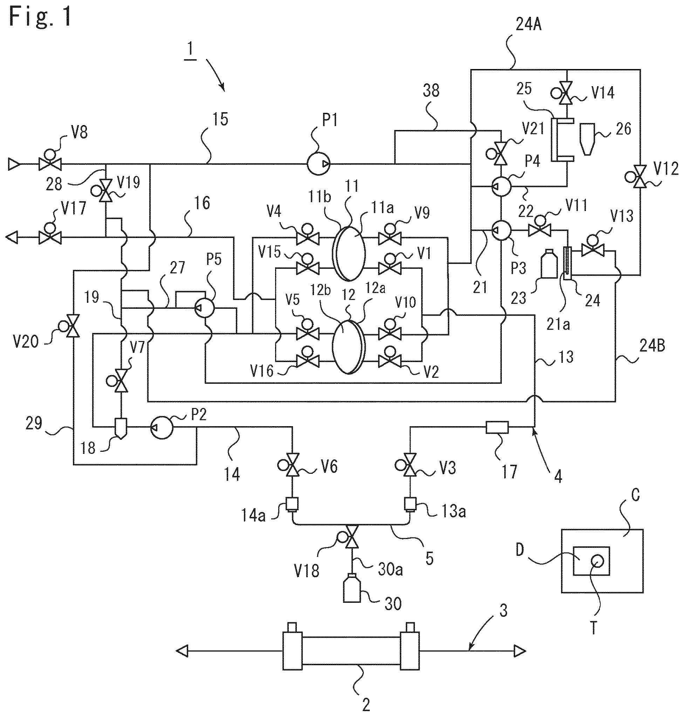

Embodiments according to the present invention will be described referring to the drawings. FIG. 1 shows a liquid circuit of a hemodialyzer 1 as the hemocatharsis apparatus for dialysis treatment. The hemodialyzer 1 is controlled by a control unit C.

The hemodialyzer 1 includes a dialyzer 2 as a hemocatharsis device for dialysis between the blood and the dialysate through diffusion and ultrafiltration, a blood circuit 3 connected to the dialyzer 2 for distribution of the blood, and a dialysate circuit 4 connected to the dialyzer 2 for distribution of the dialysate.

The hemodialyzer 1 according to the embodiment is configured to clean the dialysate circuit 4 after termination of the dialysis treatment. FIG. 1 shows a circuit structure for the cleaning operation, in which the dialysate circuit 4 is disconnected from the dialyzer 2, and connected to a connection passage 5.

The dialysate circuit 4 includes two identical metering containers, that is, a first metering chamber 11 and a second metering chamber 12. Each inner part of the first and the second metering chambers 11, 12 is partitioned with a diaphragm into supply chambers 11a, 12a for containing fresh dialysate, and into recovery chambers 11b, 12b for containing used dialysate, respectively.

A dialysate supply passage 13 for distributing the fresh dialysate is connected between the supply chambers 11a, 12a of the first and the second metering chambers 11, 12, and the dialyzer 2. A dialysate recovery passage 14 for distributing the used dialysate is connected between the recovery chambers 11b, 12b and the dialyzer 2.

A water supply passage 15 communicated with a not shown purified water supply unit for supplying the purified water is connected to the supply chambers 11a, 12a of the first and the second metering chambers 11, 12. A draining passage 16 for discharging the used dialysate to a not shown waste liquid tank is connected to the recovery chambers 11b, 12b. The water supply passage 15 is provided with a water supply pump P1.

An upstream part of the dialysate supply passage 13 is branched into two passages connected to the supply chambers 11a, 12a of the first and the second metering chambers 11, 12, respectively. Supply valves V1, V2 to be opened and closed under the control of the control unit C are disposed in the respective branched passages.

A downstream part of the dialysate supply passage 13 is connectable to the dialyzer 2 via a connector 13a. In cleaning as indicated by FIG. 1, the connector 13a is connected to one end of the connection passage 5.

The dialysate supply passage 13 is provided with a concentration sensor 17 for measuring the liquid concentration by measurement of electro-conductivity, and a switching valve V3 to be opened and closed under the control of the control unit C at a position adjacent to the connector 13a.

A downstream part of the dialysate recovery passage 14 is branched into two passages connected to the recovery chambers 11b, 12b of the first and the second metering chambers 11, 12, respectively. Recovery valves V4, V5 to be opened and closed under the control of the control unit C are disposed in the respective branched passages.

An upstream part of the dialysate recovery passage 14 is connectable to the dialyzer 2 via a connector 14a. In cleaning as indicated by FIG. 1, the connector 14a is connected to the other end of the connection passage 5.

A switching valve V6 to be opened and closed under the control of the control unit C is provided at a position adjacent to the connector 14a of the dialysate recovery passage 14. A dialysate pump P2 for supplying the dialysate and an air removal tank 18 are provided in series at the downstream side of the switching valve V6.

The air removal tank 18 is disposed in a branch passage 19 communicated with the draining passage 16. The branch passage 19 is provided with a switching valve V7 to be opened and closed under the control of the control unit C.

A part of the water supply passage 15 at the upstream side of the water supply pump P1 is provided with a switching valve V8 to be opened and closed under the control of the control unit C. A part of the water supply passage 15 at the downstream side of the water supply pump P1 is branched into two passages connected to the supply chambers 11a, 12a, respectively. Liquid supply valves V9, V10 to be opened and closed under the control of the control unit C are disposed in the respective branch passages.

A part of the water supply passage 15 at the upstream side of the branch passages is connected to a liquid A passage 21 and a liquid B passage 22 as the undiluted dialysate passages for distributing the undiluted dialysate, that is, the liquid A and the liquid B. Undiluted dialysate metering pumps, that is, a liquid A metering pump P3 and a liquid B metering pump P4 are disposed in the liquid A passage 21 and the liquid B passage 22, respectively.

A suction member 21a to be inserted into the liquid A tank 23 that contains the liquid A is disposed at one end of the liquid A passage 21. A switching valve V11 to be opened and closed under the control of the control unit C is disposed between the suction member 21a and the liquid A metering pump P3.

In the dialysis treatment, the suction member 21a is inserted into the liquid A tank 23, and the liquid A pumped up by the liquid A metering pump P3 is supplied to the supply chambers 11a, 12a of the first and the second metering chambers 11, 12 via the water supply passage 15.

In the cleaning operation, the suction member 21a is taken out from the liquid A tank 23, and stored in a cleaning cylinder 24. A liquid supply passage 24A is connected to a lower end of the cleaning cylinder 24, and a discharge passage 24B is connected to an upper end.

An upstream end of the liquid supply passage 24A is connected to a part of the water supply passage 15 at the downstream side of the water supply pump P1, and a downstream end of the discharge passage 24B is connected to a part of the branch passage 19 closer to the draining passage 16 than the switching valve V7.

Switching valves V12, V13 to be controlled by the control unit C are disposed in the liquid supply passage 24A and the discharge passage 24B, respectively.

The liquid B passage 22 is connected to the liquid supply passage 24A at the upstream side of the switching valve V12. A dissolving unit 25 for dissolving the powder as the base material for the liquid B is disposed in the middle of the liquid B passage 22, and a switching valve V14 to be opened and closed under the control of the control unit C is disposed at the upstream side of the dissolving unit 25.

In the dialysis treatment, a cartridge 26 that contains the powder is attached to the dissolving unit 25. The purified water distributing in the water supply passage 15 from the liquid supply passage 24A is supplied into the cartridge 26 so that the powder is dissolved.

A predetermined amount of the liquid B as a result of dissolving the powder is supplied through the liquid B passage 22 by the liquid B metering pump P4 to the supply chambers 11a, 12a of the first and the second metering chambers 11, 12 via the water supply passage 15.

In the cleaning operation, the cartridge 26 is detached from the dissolving unit 25 so that the cleaning liquid is distributed through a not shown cleaning passage formed inside the dissolving unit 25.

An upstream part of the draining passage 16 is branched into two passages connected to the recovery chambers 11b, 12b of the first and the second metering chambers 11, 12, respectively. Draining valves V15, V16 to be opened and closed under the control of the control unit C are disposed in the respective branch passages. A switching valve V17 to be opened and closed under the control of the control unit C is disposed at a downstream part of the draining passage 16.

The hemodialyzer 1 of the embodiment allows removal of surplus water contained in the blood in the dialysis treatment. A water removal passage 27 for distributing the used dialysate is formed from a position of the dialysate recover passage 14 to be branched into the passages having the respective recovery valves V4, V5 to a position of the draining passage 16 between a part branched into the passages having the draining valves V15, V16, and the switching valve V17.

The water removal passage 27 is provided with a water removal metering pump P5 to be operated in the dialysis treatment, thus allowing removal of a predetermined amount of water from the dialyzer 2.

The water removal passage 27 of the embodiment is connected to the branch passage 19, and further connected to the draining passage 16 via a circulation passage 28 to be described later. It is also possible to connect the water removal passage 27 directly to the draining passage 16.

The hemodialyzer 1 according to the embodiment is configured to connect the dialysate supply passage 13 and the dialysate recovery passage 14 via the connection passage 5 for cleaning the dialysate circuit 4, and to further dispose the circulation passage 28 between the draining passage 16 and the water supply passage 15, and a cleaning liquid buffer passage 29 between the dialysate recovery passage 14 and the water supply passage 15.

A cleaning liquid tank 30 that contains the undiluted cleaning liquid mainly formed of citric acid is connected to the connection passage 5 via a passage 30a. The passage 30a is provided with a switching valve V18 to be controlled by the control unit C.

One end of the circulation passage 28 is connected to a position of the draining passage 16 at the upstream side of the switching valve V17, and the other end is connected to a position between the switching valve V8 of the water supply passage 15 and the water supply pump P1. The circulation passage 28 is provided with a switching valve V19 to be controlled by the control unit C.

One end of the cleaning liquid buffer passage 29 is connected to a position of the dialysate recovery passage 14 at the upstream side of the dialysate pump P2, and the other end is connected to a position between a connected portion of the water supply passage 15 to the circulation passage 28, and the water supply pump P1. The cleaning liquid buffer passage 29 is provided with a switching valve V20 to be controlled by the control unit C.

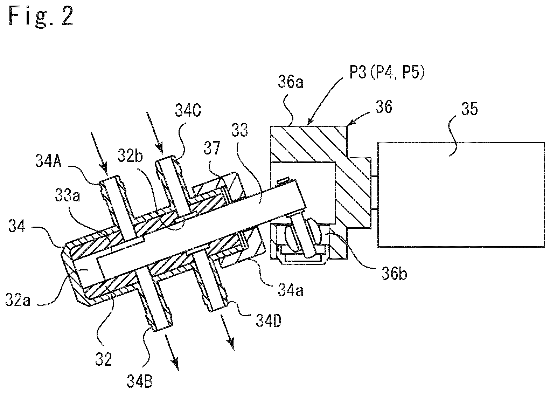

Each structure of the metering pumps P3, P4, P5 used for supplying the liquid A and liquid B, and water removal will be described referring to FIG. 2.

The piston pump called ceramic pump is used for forming the metering pump. The piston pump includes a cylindrical cylinder 32, a piston 33 that rotatingly reciprocates in the cylinder 32, a case 34 that stores the cylinder 32 and the piston 33, a motor 35 for driving the piston 33, and a coupling mechanism 36 for coupling the piston 33 with the motor 35.

The ceramic material excellent in corrosion resistance and wear resistance is used for forming the cylinder 32 and the piston 33. The cylinder 32 is stored in the cylindrical bottomed resin case 34.

Both ends of the cylinder 32 are formed in alignment with positions of the bottom and the opening of the case 34. A cap 34a is fitted to the opening of the case 34 via a resin ring seal 37.

A suction port 34A and a discharge port 34B are attached to a leading end of the case 34 at counter positions. The cylinder 32 has a through hole penetrating in alignment with positions of the suction port 34A and the discharge port 34B, and a metering chamber 32a formed therein in communication with the suction port 34A and the discharge port 34B.

Assuming that the above-described metering pumps are used for the liquid A metering pump P3 and the liquid B metering pump P4, the respective upstream parts of the liquid A passage 21 and the liquid B passage 22 are connected to the suction port 34A. Meanwhile, the respective downstream parts of those passages are connected to the discharge port 34B.

Assuming that the above-described metering pump is used for the water removal metering pump P5, the upstream part of the water removal passage 27 is connected to the suction port 34A, and the downstream part is connected to the discharge port 34B.

The piston 33 is operated by the motor 35 and the coupling mechanism 36 to reciprocally move up and down in the cylinder 32 rotatingly.

The coupling mechanism 36 includes a cylindrical member 36a fixed to a rotary shaft of the motor 35, and a spherical bearing 36b formed in an inner wall of the cylindrical member 36a. A portion of the piston 33, projecting from the cylinder 32 is connected to the spherical member of the spherical bearing 36b while penetrating therethrough.

A notch 33a is formed in the top end of the piston 33. Depending on the rotating position of the piston 33, either the suction port 34A or the discharge port 34B is closed, and the other is communicated with the metering chamber 32a.

Just before the rotating piston 33 reaches the top dead center, the suction port 34A is communicated with the metering chamber 32a via the notch 33a so that the liquid is sucked from the suction port 34A while increasing the volume.

Just before the rotating piston 33 reaches the bottom dead center, the discharge port 34B is communicated with the metering chamber 32a via the notch 33a so that the liquid of the metering chamber 32a is discharged from the discharge port 34B while decreasing the volume.

Assuming that the above-structured metering pump is used for the liquid A metering pump P3 and the liquid B metering pump P4, the small amount of the undiluted liquid flowing into the metering chamber 32a may infiltrate into the gap between the sliding portions of the inner surface of the cylinder 32 and the outer surface of the piston 33. The infiltrated liquid is crystallized to deteriorate slidability of the cylinder 32 and the piston 33.

Assuming that the above-structured metering pump is used for the water removal metering pump P5, the dialysate flowing into the metering chamber 32a may partially infiltrate into the gap. The calcium carbonate resulting from deposition of the dialysate deteriorates the slidability of the cylinder 32 and the piston 33, thus immobilizing those members.

For that reason, a flushing passage 38 is formed between the water supply passage 15 and the water removal passage 27 for washing out the liquid infiltrating into the gap in the sliding portion in the dialysis treatment with the purified water. The flushing passage 38 is connected to the liquid B metering pump P4, the liquid A metering pump P3, and the water removal metering pump P5 sequentially from the upstream side. A switching valve V21 to be controlled by the control unit C is disposed in the flushing passage 38 at the upstream side of the liquid B metering pump P4.

At a base end side of the case 34 of the metering pump, an inflow port 34C and an outflow port 34D are disposed at counter positions. The cylinder 32 has a through hole corresponding to the inflow port 34C and the outflow port 34D. A groove 32b to be communicated with those inflow port 34C and the outflow port 34D is formed in the inner circumferential surface of the cylinder 32.

The inflow port 34C is connected to the position at the upstream side of the flushing passage 38, and the outflow port 34D is connected to the position at the downstream side of the flushing passage 38.

In the structure as described above, the liquid flowing into the inflow port 34C from the flushing passage 38 is distributed through the groove 32b, and discharged from the outflow port 34D. Meanwhile, the liquid distributing through the groove 32b partially infiltrates into the gap between the inner surface of the cylinder 32 and the outer surface of the piston 33 to wash away the undiluted liquid and the dialysate infiltrated in the gap between the sliding portions of the cylinder 32 and the piston 33.

In the dialysis treatment, the above-structured dialysate circuit 4 is configured to supply the fresh dialysate prepared in the supply chambers 11a, 12a of the first and the second metering chambers 11, 12 to the dialyzer 2 alternately via the dialysate supply passage 13, and to recover the used dialysate that has been distributed through the dialyzer 2 to the recovery chambers 11b, 12b alternately via the dialysate recovery passage 14.

When the fresh dialysate is prepared in the supply chamber 11a of the first metering chamber 11, the water supply pump P1 of the water supply passage 15 supplies the purified water to the supply chamber 11a so that the liquid A metering pump P3 of the liquid A passage 21 supplies a predetermined amount of the liquid A in the liquid A tank 23 to the supply chamber 11a. Meanwhile, the liquid B metering pump P4 of the liquid B passage 22 supplies a predetermined amount of the liquid B dissolved by the dissolving unit 25 to the supply chamber 11a.

The purified water, the liquid A, and the liquid B flowing into the supply chamber 11a of the first metering chamber 11 are mixed at a predetermined rate so that the fresh dialysate is prepared in the supply chamber 11a. Accompanying with the operation, the diaphragm of the first metering chamber 11 is deformed to push the used dialysate that has been recovered in the recovery chamber 11b out to the draining passage 16.

Meanwhile, in the second metering chamber 12, the dialysate pump P2 of the dialysate recovery passage 14 allows inflow of the used dialysate in the dialyzer 2 to the recovery chamber 12b. As a result, the diaphragm in the second metering chamber 12 is deformed to push the fresh dialysate that has been prepared in the supply chamber 12a out to the dialysate supply passage 13.

Upon water removal in the dialysis treatment, the water removal metering pump P5 is activated to discharge the predetermined amount of the used dialysate from the draining passage 16 via the water removal passage 27.

For a required time in the dialysis treatment, the purified water from the water supply passage 15 is distributed to the flushing passage 38. As a result, the liquid A metering pump P3, the liquid B metering pump P4, and the water removal metering pump P5 are operated to clean the sliding portions of the cylinder 32 and the piston 33 using the purified water flowing from the inflow port 34C.

After termination of the hemodialysis treatment using the hemodialyzer 1 as described above, the dialyzer 2 is disconnected from the dialysate circuit 4 so that the respective passages, pumps, and the switching valves which constitute the dialysate circuit 4 are cleaned with the cleaning liquid.

The cleaning liquid for cleaning the dialysate circuit 4 contains citric acid. The use of the high-concentration undiluted cleaning liquid to be distributed to the liquid A metering pump P3, the liquid B metering pump P4, and the water removal metering pump P5 may cause the risk of corroding the ceramic cylinders 32 and the ceramic pistons 33 which constitute those pumps owing to the high concentration citric acid.

The hemodialyzer 1 according to the embodiment is configured to circulate the undiluted cleaning liquid together with the purified water through the closed circuit formed in the dialysate circuit 4 to prepare the sufficiently diluted cleaning liquid with predetermined concentration. Then the resultant cleaning liquid is used for cleaning the liquid A metering pump P3, the liquid B metering pump P4, and the water removal metering pump P5.

Specifically, after terminating the hemodialysis treatment, the dialysate supply passage 13 and the dialysate recovery passage 14 are disconnected from the dialyzer 2, and the connectors 13a, 14a at the respective ends of those passages are connected to both ends of the connection passage 5.

In the liquid A passage 21, the suction member 21a is detached from the liquid A tank 23, and inserted into the cleaning cylinder 24. Meanwhile, in the liquid B passage 22, the cartridge 26 is detached from the dissolving unit 25.

Upon start of the cleaning operation in the above-described state via the control unit C, the control unit C activates the water supply pump P1 and the dialysate pump P2 while closing the switching valve V18 in the passage 30a connected to the cleaning liquid tank 30. Then the purified water is supplied from the water supply passage 15, and is drained from the draining passage 16 so that the purified water is distributed to all the passages constituting the dialysate circuit 4 for replacing the dialysate and the like in those passages with the purified water.

In the dialysis treatment, the purified water is also distributed to the branch passage 19 connected to the air removal tank 18 for air distribution so that the branch passage 19 is filled with the purified water.

When the dialysate circuit 4 is filled with the purified water, the control unit C executes the preparatory process for drawing the undiluted cleaning liquid by closing the liquid supply valves V9, V10 connected to the supply chambers 11a, 12a of the first and the second metering chambers 11, 12 while opening the supply valves V1, V2, and opening the recovery valves V4, V5 connected to the recovery chambers 11b, 12b while closing the draining valves V15, V16.

As the liquid is supplied by the dialysate pump P2, the purified water is discharged from the supply chambers 11a, 12a of the first and the second metering chambers 11, 12 to the dialysate supply passage 13 to minimize the volume. The purified water is flown into the recovery chambers 11b, 12b from the dialysate recovery passage 14 to maximize the volume.

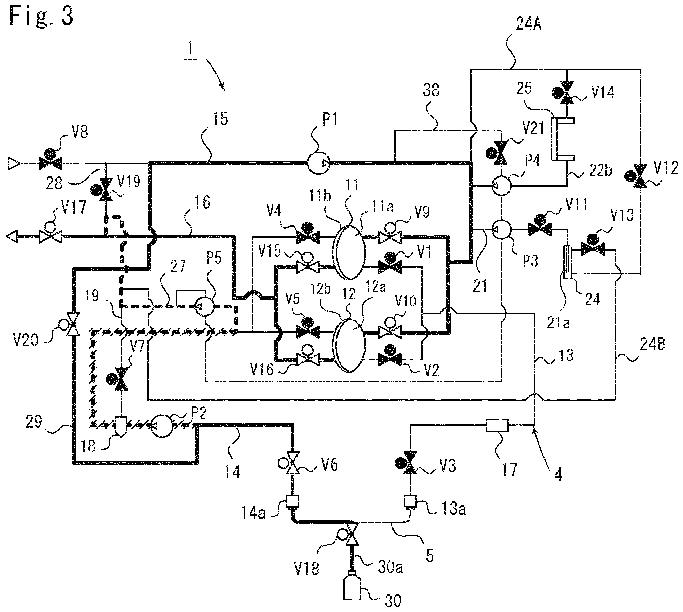

FIG. 3 represents the operation for drawing the high concentration undiluted cleaning liquid contained in the cleaning liquid tank 30 to the dialysate circuit 4. Referring to the drawing, the black-colored switching valves are in the closed states, and the blank switching valves are in the opened states.

The control unit C closes the switching valve V8 of the water supply passage 15, the switching valve V19 of the circulation passage 28, the switching valve V11 of the liquid A passage 21, the switching valve V12 of the liquid supply passage 24A, the switching valve V13 of the discharge passage 24B, the switching valve V14 of the liquid B passage 22, and the switching valve V21 of the flushing passage 38.

The control unit C opens the liquid supply valves V9, V10 connected to the supply chambers 11a, 12a of the first and the second metering chambers 11, 12, while closing the supply valves V1, V2, closes the recovery valves V4, V5 connected to the recovery chambers 11b, 12b while opening the draining valves V15, V16, and further closes the switching valve V3 of the dialysate supply passage 13.

From the above-described state, the control unit C activates the water supply pump P1 after opening the switching valve V18 of the passage 30a connected to the cleaning liquid tank 30, and the switching valve V20 of the cleaning liquid buffer passage 29.

As a result, the purified water flows into the supply chambers 11a, 12a of the first and the second metering chambers 11, 12 from the water supply passage 15 so that the purified water by the amount equivalent to the inflow amount is discharged outside from the recovery chambers 11b, 12b via the draining passage 16.

As the switching valve V8 of the water supply passage 15, and the switching valve V19 of the circulation passage 28 are closed, the purified water flowing through the cleaning liquid buffer passage 29 and the dialysate recovery passage 14 is drawn to the water supply passage 15.

Accompanying with the operation as described above, the undiluted cleaning liquid is drawn from the cleaning liquid tank 30 via the connection passage 5, and distributed through the dialysate recovery passage 14 and the cleaning liquid buffer passage 29. The undiluted cleaning liquid finally flows into the water supply passage 15 (see the section indicated by the bold line in FIG. 3).

As the purified water is contained in the supply chambers 11a, 12a of the first and the second metering chambers 11, 12, the amount of the undiluted cleaning liquid to be drawn may be obtained so that the predetermined amount of the undiluted cleaning liquid is supplied to the dialysate circuit 4.

After drawing the predetermined amount of the undiluted cleaning liquid to the dialysate circuit 4, the control unit C closes the switching valve V20 of the cleaning liquid buffer passage 29 so that the water removal metering pump P5 of the water removal passage 27 is activated.

Accordingly, the predetermined amount of purified water is discharged from the dialysate recovery passage 14 to the draining passage 16 via the water removal passage 27. Accompanying with the operation, the predetermined amount of undiluted cleaning liquid is drawn from the cleaning liquid tank 30 to the dialysate recovery passage 14 via the connection passage 5 (see the section indicated by the bold broken line in FIG. 3).

The amount of the purified water to be supplied by the water removal metering pump P5 is set so that the flow of the drawn undiluted cleaning liquid stops just before the position of the dialysate recovery passage 14 connected to the water removal passage 27 (see the section with hatching indicated by bold broken line in FIG. 3).

In the operations as described above, the required amount of the undiluted cleaning liquid is measured by the first and the second metering chambers 11, 12, and the water removal metering pump P5 to allow the measured amount of the undiluted cleaning liquid to be drawn from the cleaning liquid tank 30 to the dialysate circuit 4.

However, the undiluted cleaning liquid drawn from the cleaning liquid tank 30 is kept highly concentrated while being insufficiently diluted with the purified water. Distribution of the high-concentration undiluted cleaning liquid to the liquid A metering pump P3, the liquid B metering pump P4, and the water removal metering pump P5 may cause the risk of corroding the ceramic members constituting those pumps.

In the embodiment, the undiluted cleaning liquid is kept from coming in contact with the liquid A metering pump P3, the liquid B metering pump P4, and the water removal metering pump P5 when supplying the liquid into the dialysate circuit 4. The undiluted cleaning liquid is then circulated together with the purified water for dilution to have the predetermined concentration.

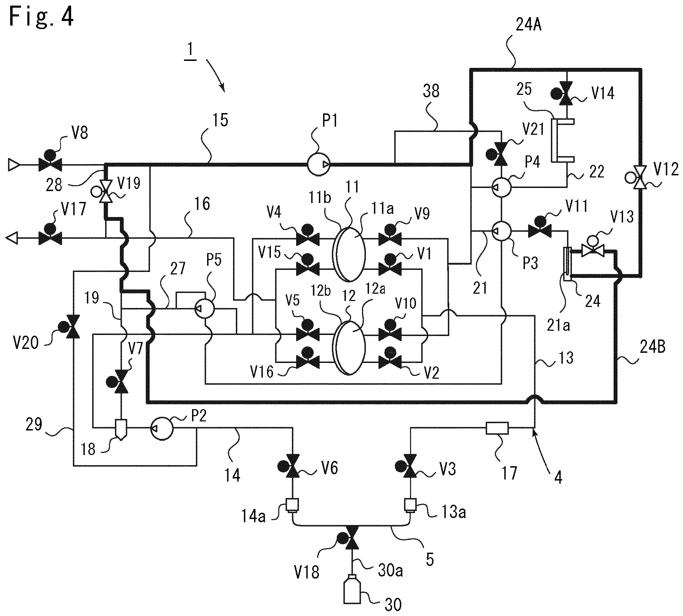

In the embodiment, as FIG. 4 shows, from the state in which the predetermined amount of the undiluted cleaning liquid has been drawn into the dialysate circuit 4, the control unit C controls to close the switching valve V20 of the cleaning liquid buffer passage 29, the switching valve V6 of the dialysate recovery passage 14, the switching valve V18 of the passage 30a connected to the cleaning liquid tank 30, the switching valve V17 of the draining passage 16, the liquid supply valves V9, V10 of the water supply passage 15, and the draining valves V15, V16 of the draining passage 16 while keeping the switching valve V8 of the water supply passage 15, the switching valve V3 of the dialysate supply passage 13, the switching valve V11 of the liquid A passage 21, the switching valve V14 of the liquid B passage 22, and the switching valve V21 of the flushing passage 38 closed. Then the control unit C further controls to open the switching valve V19 of the circulation passage 28, the switching valve V12 of the liquid supply passage 24A, and the switching valve V13 of the discharge passage 24B.

The control operation as described above forms the closed circuit (see the section indicated by the bold line in FIG. 4) starting from the water supply passage 15 via the liquid supply passage 24A and the discharge passage 24B both connected to the cleaning cylinder 24, and returning to the water supply passage 15.

In the state as described above, the control unit C activates the water supply pump P1 to circulate the undiluted cleaning liquid that has been drawn into the water supply passage 15 through the closed circuit, while having the liquid stirred and diluted with the purified water in the liquid supply passage 24A and the discharge passage 24B.

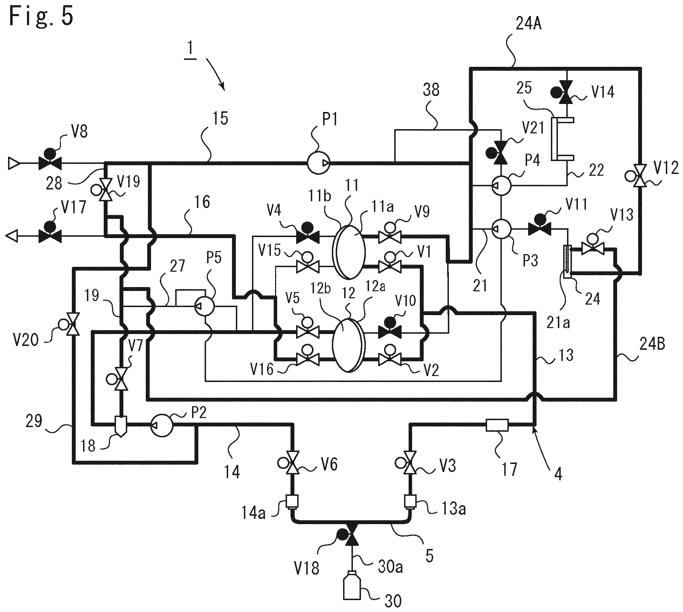

As FIG. 5 shows, the control unit C opens the switching valve V20 of the cleaning liquid buffer passage 29, the switching valve V7 of the branch passage 19, the switching valve V6 of the dialysate recovery passage 14, and the switching valve V3 of the dialysate supply passage 13, and further opens the supply valves V1, V2 of the dialysate supply passage 13, the liquid supply valve V9 of the water supply passage 15, the recovery valve V5 of the dialysate recovery passage 14, and the draining valves V15, V16 of the draining passage 16.

Operating those valves allows inflow of the undiluted cleaning liquid in the cleaning liquid buffer passage 29 into the water supply passage 15. The closed circuit is formed, in which the undiluted cleaning liquid is distributed from the water supply passage 15 to the dialysate supply passage 13, the connection passage 5, and the dialysate recovery passage 14 via the supply chamber 11a of the first metering chamber 11, and returned to the water supply passage 15 after distributing through the draining passage 16 and the circulation passage 28 via the recovery chamber 12b of the second metering chamber 12. In the above-described state, the liquid may be distributed to the branch passage 19 (see the section indicated by the bold line in FIG. 5).

The control unit C activates the dialysate pump P2 in addition to the water supply pump P1 in the closed circuit.

The water supply pump P1 serves to distribute the undiluted cleaning liquid in the cleaning liquid buffer passage 29 from the water supply passage 15 to the dialysate supply passage 13 via the supply chamber 11a, and allows the undiluted cleaning liquid to reach the dialysate recovery passage 14 via the connection passage 5.

Meanwhile, the dialysate pump P2 serves to distribute the undiluted cleaning liquid in the dialysate recovery passage 14 through the draining passage 16 via the recovery chamber 12b. The undiluted cleaning liquid partially distributes through the branch passage 19 to flow into the water supply passage 15 from the circulation passage 28.

As described above, the undiluted cleaning liquid drawn into the cleaning liquid buffer passage 29 and the dialysate recovery passage 14 is stirred and diluted with the purified water while distributing through the supply chamber 11a of the first metering chamber 11, the dialysate supply passage 13, the dialysate recovery passage 14, the recovery chamber 12b of the second metering chamber 12, the draining passage 16, the branch passage 19, and the circulation passage 28.

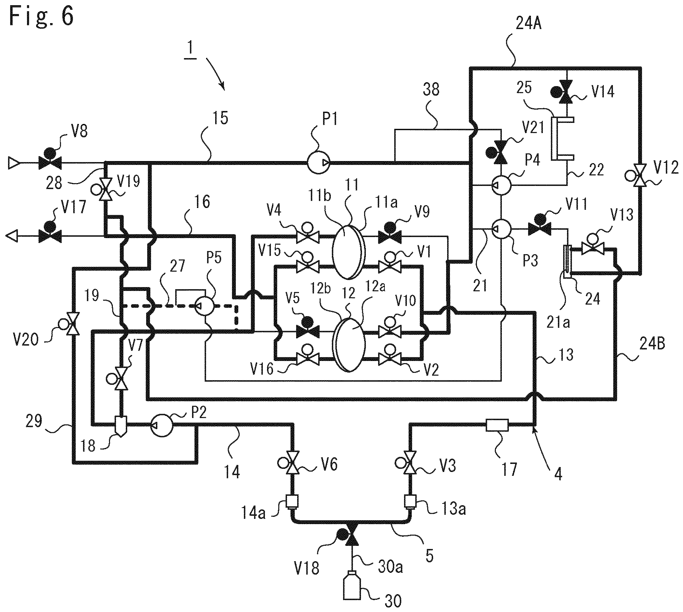

After an elapse of a predetermined period of time, the control unit C closes the liquid supply valve V9 of the water supply passage 15 while opening the liquid supply valve V10 as shown in FIG. 6 to allow passage of the liquid through the supply chamber 12a of the second metering chamber 12. The control unit C further closes the recovery valve V5 of the dialysate recovery passage 14 while opening the recovery valve V4 to allow passage of the liquid through the recovery chamber 11b of the first metering chamber 11.

The undiluted cleaning liquid and the purified water distributing through the water supply passage 15 is allowed by the water supply pump P1 to distribute through the dialysate supply passage 13 via the supply chamber 12a of the second metering chamber 12. Meanwhile, the dialysate recovery passage 14 allows distribution of the liquid through the draining passage 16 by the dialysate pump P2 via the recovery chamber 11b of the first metering chamber 11.

Thereafter, the control unit C switches between the state as shown in FIG. 5 and the state as shown in FIG. 6 alternately to allow the liquid to pass through the supply chamber 11a and the recovery chamber 11b of the first metering chamber 11, and the supply chamber 12a and the recovery chamber 12b of the second metering chamber 12 at a predetermined time interval alternately.

When the liquid is allowed to pass through the supply chamber 11a of the first metering chamber 11 or the supply chamber 12a of the second metering chamber 12, the draining valve V15 or V16 is opened to allow the liquid to be discharged from the recovery chamber 11b or 12b at the counter side.

When the liquid is allowed to pass through the recovery chamber 11b of the first metering chamber 11 or the recovery chamber 12b of the second metering chamber 12, the supply valve V1 or V2 is opened to allow the liquid to be discharged from the supply chamber 11a or 12a at the counter side.

The first closed circuit (see the sections indicated by bold lines in FIG. 5 and FIG. 6) is configured to stir the purified water and the undiluted cleaning liquid in circulation so that the undiluted cleaning liquid is diluted to have the predetermined concentration. Accompanying with the operation, the first closed circuit is cleaned.

The control unit C includes a determination unit D that determines whether the concentration of the cleaning liquid is appropriate. The determination unit D includes a timer T. In the case of elapsing the predetermined time for sufficient dilution of the undiluted cleaning liquid from the activation of the dialysate pump P2 in the first closed circuit as shown in FIG. 5 or FIG. 6, it is determined that the undiluted cleaning liquid has been sufficiently diluted to have the predetermined concentration.

When the determination unit D determines that the undiluted cleaning liquid has been diluted to have the predetermined concentration, the control unit C activates the water removal metering pump P5 to distribute the cleaning liquid that has been diluted to have the predetermined concentration to the water removal passage 27 (see the section indicated by the bold broken line in FIG. 6).

The control unit C operates the water removal metering pump P5 for a predetermined time required for cleaning the water removal passage 27. This makes it possible to clean the water removal passage 27, and the metering chamber 32a in the cylinder 32 of the water removal metering pump P5 using the cleaning liquid.

The determination unit D may be configured to allow the concentration sensor 17 of the dialysate supply passage 13 to measure the concentration (electro-conductivity) of the purified water and the undiluted cleaning liquid in circulation in the first closed circuit to determine that the undiluted cleaning liquid has been diluted to have the predetermined concentration.

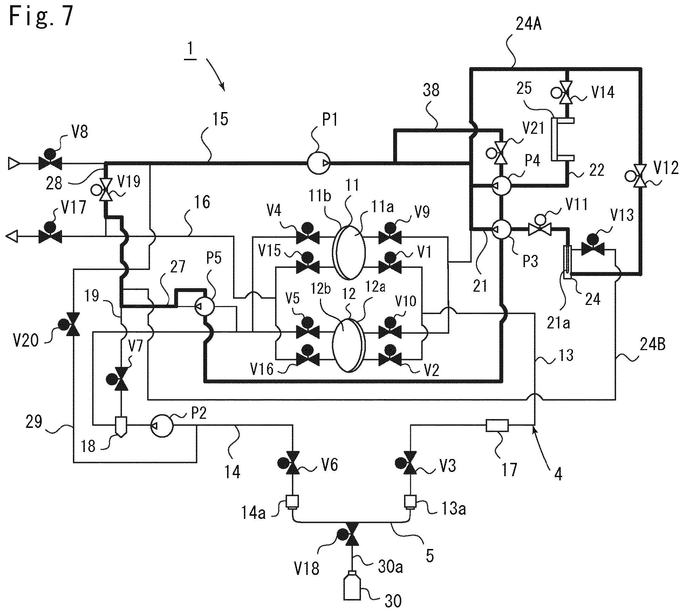

Referring to FIG. 7, in operation of the water removal metering pump P5 for a predetermined time for cleaning the water removal metering pump P5 and the water removal passage 27, the control unit C closes the switching valve V20 of the cleaning liquid buffer passage 29, the switching valve V7 of the branch passage 19, the switching valve V6 of the dialysate recovery passage 14, the switching valve V3 of the dialysate supply passage 13, and the switching valve V13 of the discharge passage 24B of the cleaning cylinder 24 while closing all valves in the first and the second metering chambers, that is, the liquid supply valves V9, V10, and supply valves V1, V2, which are connected to the supply chambers 11a, 12a of the first and the second metering chambers 11, 12, and the recovery valves V4, V5, and the draining valves V15, V16, which are connected to the recovery chambers 11b, 12b. The control unit C controls to stop operations of the dialysate pump P2 and the water removal metering pump P5.

The control unit C forms a second closed circuit (see the section indicated by bold line in FIG. 7) by opening the switching valve V14 of the liquid B passage 22, and the switching valve V11 of the liquid A passage 21 so that the liquid B metering pump P4 and the liquid A metering pump P3 are activated in addition to the water supply pump P1.

As a result, the cleaning liquid that has been diluted to have the predetermined concentration flows from the water supply passage 15 to the flushing passage 38, and circulates to the water supply passage 15 via the branch passage 19 and the circulation passage 28.

Meanwhile, the cleaning liquid flowing through the liquid supply passage 24A partially flows into the liquid B passage 22 to pass through the inner passage of the dissolving unit 25. It is then pushed by the liquid B metering pump P4 out to the water supply passage 15, and circulated to the liquid supply passage 24A.

The cleaning liquid remaining in the liquid supply passage 24A flows into the cleaning cylinder 24, and then distributes from the suction member 21a to the liquid A passage 21. The cleaning liquid is then pushed by the liquid A metering pump P3 out to the water supply passage 15, and circulated to the liquid supply passage 24A.

As the control unit C circulates the cleaning liquid through the second closed circuit for the predetermined time required for cleaning, the liquid A passage 21, the liquid B passage 22, and the metering chambers 32a formed inside the respective cylinders 32 constituting the liquid A metering pump P3, and the liquid B metering pump P4. At the same time, it is possible to clean the flushing passage 38, and the grooves 32b formed in the respective cylinders 32 of the liquid B metering pump P4 and the liquid A metering pump P3.

As described above, the cleaning liquid distributing through the second closed circuit has been already diluted to have the predetermined concentration. Even if the cleaning liquid distributes through the metering pump formed of the ceramic members, corrosion in those members may be suppressed. Furthermore, those members may be cleaned with the cleaning liquid with appropriate concentration.

After distributing the cleaning liquid through the first and the second closed circuits for the predetermined time, the control unit C stops circulation of the cleaning liquid, and allows distribution of the purified water to the passages constituting the dialysate circuit 4 for replacement of the cleaning liquid with the purified water. The cleaning operation of the dialysate circuit 4 is terminated.

In the embodiment as described above, when cleaning the dialysate circuit 4 with the cleaning liquid, the undiluted cleaning liquid is drawn into the dialysate circuit 4 as shown in FIG. 3. In the above-described state, the liquid A metering pump P3, the liquid B metering pump P4, and the water removal metering pump P5 are kept from being in contact with the high-concentration undiluted cleaning liquid.

Furthermore, as FIG. 5 and FIG. 6 show, the undiluted cleaning liquid is circulated through the first closed circuit until it is diluted with the purified water to have the predetermined concentration. The high-concentration undiluted cleaning liquid is kept from distributing through the liquid A metering pump P3, the liquid B metering pump P4, and the water removal metering pump P5 to prevent damage to the ceramic members owing to the high-concentration undiluted cleaning liquid.

After the undiluted cleaning liquid is diluted to the predetermined concentration, the liquid A metering pump P3, the liquid B metering pump P4, and the water removal metering pump P5 may be cleaned with the cleaning liquid with the predetermined concentration, circulating through the first and the second closed circuits as shown in FIG. 6 and FIG. 7.

In the first embodiment, the connection passage 5 is provided with the cleaning liquid tank 30 that contains the undiluted cleaning liquid, the passage 30a, and the switching valve V18. In the second embodiment, the cleaning liquid buffer passage 29 may be provided with those components.

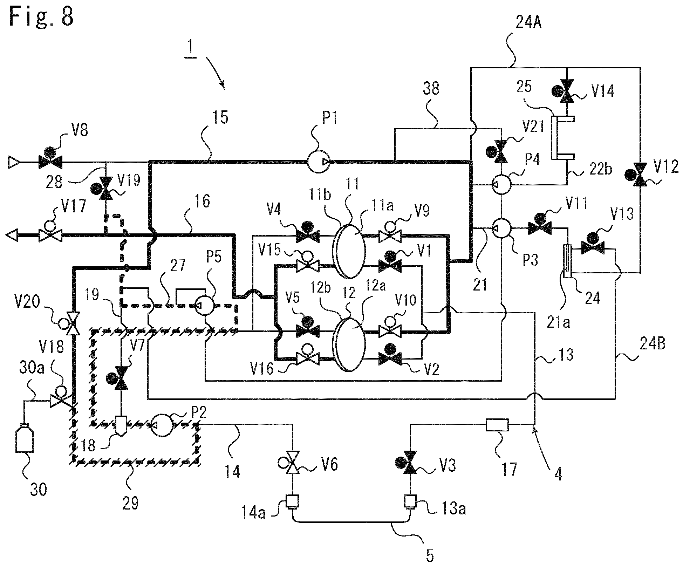

Specifically, the structure as shown in FIG. 8 is different from the one as shown in FIG. 3 in that the passage 30a of the cleaning liquid tank 30 is connected between the switching valve V20 and an end of the cleaning liquid buffer passage 29 connected to the dialysate recovery passage 14, and the passage 30a is provided with the switching valve V18.

In the above-described structure, the water supply pump P1 is activated while having the switching valve V6 of the dialysate recovery passage 14 closed. This makes it possible to draw the undiluted cleaning liquid from the cleaning liquid tank 30 to the cleaning liquid buffer passage 29, and to supply the undiluted cleaning liquid to the water supply passage 15 likewise the first embodiment.

Activation of the water removal metering pump P5 by closing the switching valve V20 of the cleaning liquid buffer passage 29 allows drawing of the undiluted cleaning liquid from the cleaning liquid buffer passage 29 to the portion before the position of the dialysate recovery passage 14 connected to the water removal passage 27.

In the state as descried above, likewise the first embodiment, the undiluted cleaning liquid is diluted to have the predetermined concentration in the first closed circuit. The second closed circuit is formed sequentially to allow cleaning of the dialysate circuit 4.

In this embodiment, the ceramic pumps are used for the metering pumps, and the undiluted cleaning liquid contains citric acid. Assuming that any other material is used for forming the metering pump, if the undiluted cleaning liquid has the component that may damage the members constituting the metering pump, the procedures as described above may be executed to prevent the damage to the metering pump.

### Reference Signs List

- 1: hemodialyzer (hemocatharsis apparatus)
- 2: dialyzer (hemocatharsis device)
- 4: dialysate circuit
- 5: connection passage
- 11: first metering chamber (metering container)
- 12: second metering chamber (metering container)
- 13: dialysate supply passage
- 14: dialysate recovery passage
- 15: water supply passage
- 16: draining passage
- 21: liquid A passage (undiluted dialysate passage)
- 22: liquid B passage (undiluted dialysate passage)
- 27: water removal passage
- 28: circulation passage
- 38: flushing passage
- P3: liquid A metering pump (metering pump for the undiluted liquid)
- P4: liquid B metering pump (metering pump for the undiluted liquid)
- P5: water removal metering pump

## Claims

1. A hemocatharsis apparatus (1) comprising:
a metering container (11, 12) having a supply chamber for containing a fresh dialysate, and a recovery chamber for containing a used dialysate;
a dialysate supply passage (13) for supplying the fresh dialysate from the supply chamber to a hemocatharsis device (2);
a dialysate recovery passage (14) for recovering the used dialysate from the hemocatharsis device (2) to the recovery chamber;
a dialysate pump (P2) disposed in the dialysate recovery passage (14);
a water supply passage (15) for supplying purified water to the supply chamber;
a water supply pump (P1) disposed in the water supply passage (15);
a draining passage (16) for draining the used dialysate from the recovery chamber;
a water removal passage (27) for connecting the dialysate recovery passage (14) and the draining passage (16) while bypassing the metering container (11, 12);
a water removal metering pump (P5) disposed in the water removal passage (27);
a control means (C) for controlling operations of the water removal metering pump (P5), the water supply pump (P1) and the dialysate pump (P2);
a connection passage (5) for connecting a downstream side of the dialysate supply passage (13) and an upstream side of the dialysate recovery passage (14) while bypassing the hemocatharsis device (2);
a circulation passage (28) for connecting an upstream side of the water supply passage (15) and a downstream side of the draining passage (16), the apparatus being configured such that a closed circuit is formed of the connection passage (5) and the circulation passage (28) at the time of cleaning a dialysate circuit (4) constituted by the respective passages, and a cleaning liquid is distributed to the closed circuit for cleaning the dialysate circuit;
a cleaning liquid tank (30) disposed for supply of an undiluted cleaning liquid to the dialysate circuit;
**characterized in that**:
the hemocatharsis apparatus is configured such that in a state in which the water removal metering pump (P5) is stopped, the control means (C) is configured to operate the water supply pump (P1) such that a predetermined amount of the undiluted cleaning liquid is drawn into the closed circuit from the cleaning liquid tank (30), and is further configured to operate the water supply pump (P1) and the dialysate pump (P2) to circulate purified water and the undiluted cleaning liquid through the closed circuit such that the cleaning liquid is prepared by diluting the undiluted cleaning liquid with the purified water; and
the hemocatharsis apparatus further comprises a determination unit (D) configured to determine whether a concentration of the cleaning liquid in the closed circuit is appropriate and wherein the apparatus is configured such that when it is determined by the determination unit (D) that the concentration of the cleaning liquid in the closed circuit is appropriate, the water removal metering pump (P5) is activated to distribute the cleaning liquid to the water removal passage (27) and the water removal metering pump (P5).

2. The hemocatharsis apparatus (1) according to claim 1, wherein the determination unit (D) includes a timer (T) that counts a time taken for circulating the purified water and the undiluted cleaning liquid, and the determination unit (D) is configured to determine that the concentration of the cleaning liquid in the closed circuit becomes appropriate when the counted circulation time reaches a predetermined time.

3. The hemocatharsis apparatus (1) according to claim 1, wherein the determination unit (D) includes a concentration sensor (17), and the determination unit is configured to determine that the concentration of the cleaning liquid in the closed circuit is appropriate when it is recognized that the undiluted cleaning liquid circulating through the closed circuit has been diluted to have the predetermined concentration.

4. The hemocatharsis apparatus (1) according to any one of claims 1 to 3, further comprising an undiluted dialysate passage (21, 22) connected to the water supply passage (15) for supplying an undiluted dialysate, and a metering pump (P3, P4) for the undiluted dialysate in the undiluted dialysate passage (21, 22), wherein the apparatus is configured such that when it is determined by the determination unit (D) that the concentration of the cleaning liquid in the closed circuit is appropriate, the metering pump for the undiluted dialysate (P3, P4) is activated to distribute the cleaning liquid to the undiluted dialysate passage (21, 22) and the metering pump for the undiluted dialysate (P3, P4).

5. The hemocatharsis apparatus (1) according to claim 4, wherein:
each of the water removal metering pump (P5) and the metering pump for the undiluted dialysate (P3, P4) includes a cylindrical cylinder and a piston that moves reciprocally within the cylinder;
a flushing passage (38) is disposed for supplying the purified water to a sliding portion between the piston and the cylinder; and
the apparatus is configured such that when it is determined by the determination unit (D) that the concentration of the cleaning liquid in the closed circuit is appropriate, the cleaning liquid is distributed through the flushing passage (38).

## Patentansprüche

1. Hämodialysevorrichtung (1), die Folgendes umfasst:
einen Dosierbehälter (11, 12), der eine Zufuhrkammer, die frisches Dialysat enthält, und eine Rückgewinnungskammer, die gebrauchtes Dialysat enthält, umfasst;
einen Dialysatzufuhrkanal (13), um das frische Dialysat von der Zufuhrkammer zu einer Hämodialysevorrichtung (2) zuzuführen;
einen Dialysatrückgewinnungskanal (14), um das gebrauchte Dialysat von der Hämodialysevorrichtung (2) in die Rückgewinnungskammer zurückzugewinnen;
eine Dialysatpumpe (P2), die in dem Dialysatrückgewinnungskanal (14) angeordnet ist;
einen Wasserzufuhrkanal (15), um gereinigtes Wasser zu der Zufuhrkammer zuzuführen;
eine Wasserzufuhrpumpe (P1), die in dem Wasserzufuhrkanal (15) angeordnet ist;
einen Ableitungskanal (16), um das gebrauchte Dialysat aus der Rückgewinnungskammer abzuleiten;
einen Wasserabscheidungskanal (27), um den Dialysatrückgewinnungskanal (14) mit dem Ableitungskanal (16) zu verbinden, wobei der Dosierbehälter (11, 12) umgangen wird;
eine Wasserabscheidungsdosierpumpe (P5), die in dem Wasserabscheidungskanal (27) angeordnet ist;
ein Steuermittel (C), um den Betrieb der Wasserabscheidungspumpe (P5), der Wasserzufuhrpumpe (P1) und der Dialysatpumpe (P2) zu steuern;
einen Verbindungskanal (5), um eine nachgelagerte Seite des Dialysatzufuhrkanals (13) und eine vorgelagerte Seite des Dialysatrückgewinnungskanals (14) zu verbinden, wobei die Hämodialysevorrichtung (2) umgangen wird;
einen Zirkulationskanal (28), um eine vorgelagerte Seite des Wasserzufuhrkanals (15) und eine nachgelagerte Seite des Ableitungskanals (16) zu verbinden, wobei die Vorrichtung so ausgelegt ist, dass ein geschlossener Kreislauf des Verbindungskanals (5) und des Zirkulationskanals (28) zum Zeitpunkt der Reinigung eines Dialysatkreislaufs (4) ausgebildet wird, der aus den entsprechenden Kanälen besteht, und eine Reinigungsflüssigkeit zur Reinigung des Dialysatkreislaufs in dem geschlossenen Kreislauf verteilt wird;
einen Reinigungsflüssigkeitstank (30), der zur Zufuhr einer unverdünnten Reinigungsflüssigkeit in den Dialysatkreislauf angeordnet ist;
**dadurch gekennzeichnet, dass**:
die Hämodialysevorrichtung so ausgelegt ist, dass das Steuermittel (C) in einem Zustand, in dem die Wasserabscheidungsdosierpumpe (P5) angehalten ist, ausgelegt ist, um die Wasserzufuhrpumpe (P1) so zu betreiben, dass eine vorbestimmte Menge der unverdünnten Reinigungsflüssigkeit aus dem Reinigungsflüssigkeitstank (30) in den geschlossenen Kreislauf gezogen wird, und die außerdem ausgelegt ist, um die Wasserzufuhrpumpe (P1) und die Dialysatpumpe (P2) zu betreiben, um gereinigtes Wasser und die unverdünnte Reinigungsflüssigkeit durch den geschlossenen Kreislauf zu zirkulieren, sodass die Reinigungsflüssigkeit durch das Verdünnen der unverdünnten Reinigungsflüssigkeit mit dem gereinigten Wasser vorbereitet wird; und
die Hämodialysevorrichtung außerdem eine Bestimmungseinheit (D) umfasst, die zur Bestimmung ausgelegt ist, ob eine Konzentration der Reinigungsflüssigkeit in dem geschlossenen Kreislauf geeignet ist, und wobei die Vorrichtung so ausgelegt ist, dass, wenn von der Bestimmungseinheit (D) bestimmt wird, dass die Konzentration der Reinigungsflüssigkeit in dem geschlossenen Kreislauf geeignet ist, die Wasserabscheidungsdosierpumpe (P5) aktiviert wird, um die Reinigungsflüssigkeit zu dem Wasserabscheidungskanal (27) und der Wasserabscheidungsdosierpumpe (P5) zu verteilen.

2. Hämodialysevorrichtung (1) nach Anspruch 1, wobei die Bestimmungseinheit (D) einen Zeitgeber (T) umfasst, der die Dauer erfasst, die zum Zirkulieren des gereinigten Wassers und der unverdünnten Reinigungsflüssigkeit benötigt wird, und die Bestimmungseinheit (D) ausgelegt ist, um zu bestimmen, dass die Konzentration der Reinigungsflüssigkeit in dem geschlossenen Kreislauf geeignet ist, wenn die erfasste Zirkulationsdauer eine vorbestimmte Dauer erreicht.

3. Hämodialysevorrichtung (1) nach Anspruch 1, wobei die Bestimmungseinheit (D) einen Konzentrationssensor (17) umfasst, und die Bestimmungseinheit ausgelegt ist, um zu bestimmen, dass die Konzentration der Reinigungsflüssigkeit in dem geschlossenen Kreislauf geeignet ist, wenn erkannt wird, dass die unverdünnte Reinigungsflüssigkeit, die durch den geschlossen Kreislauf zirkuliert, auf die vorbestimmte Konzentration verdünnt wurde.

4. Hämodialysevorrichtung (1) nach einem der Ansprüche 1 bis 3, das außerdem einen Kanal für unverdünntes Dialysat (21, 22), der mit dem Wasserzufuhrkanal (15) zum Zuführen eines unverdünnten Dialysats verbunden ist, und eine Dosierpumpe (P3, P4) für das unverdünnte Dialysat in dem Kanal für unverdünntes Dialysat (21, 22) umfasst, wobei die Vorrichtung so ausgelegt ist, dass, wenn von der Bestimmungseinheit (D) bestimmt wurde, dass die Konzentration in der Reinigungsflüssigkeit in dem geschlossenen Kreislauf geeignet ist, die Dosierpumpe für das unverdünnte Dialysat (P3, P4) aktiviert wird, um die Reinigungsflüssigkeit in den unverdünnten Dialysatkanal (21, 22) und die Dosierpumpe für das unverdünnte Dialysat (P3, P4) zu verteilen.

5. Hämodialysevorrichtung (1) nach Anspruch 4, wobei:
die Wasserabscheidungsdosierpumpe (P5) und die Dosierpumpe für das unverdünnte Dialysat (P3, P4) jeweils einen zylinderförmigen Zylinder und einen Kolben umfassen, der sich in dem Zylinder hin- und herbewegt;
ein Spülkanal (38) zum Zuführen des gereinigten Wassers zu einem Gleitabschnitt zwischen dem Kolben und dem Zylinder angeordnet ist; und
die Vorrichtung so ausgelegt ist, dass, wenn von der Bestimmungseinheit (D) bestimmt wurde, dass die Konzentration der Reinigungsflüssigkeit in dem geschlossenen Kreislauf geeignet ist, die Reinigungsflüssigkeit durch den Spülkanal (38) verteilt wird.

## Revendications

1. Appareil d'hémo-catharsis (1) comprenant :
un récipient doseur (11, 12) ayant une chambre d'alimentation pour contenir un dialysat frais, et une chambre de récupération pour contenir un dialysat utilisé ;
un passage d'alimentation en dialysat (13) pour fournir le dialysat frais de la chambre d'alimentation à un dispositif d'hémo-catharsis (2) ;
un passage de récupération de dialysat (14) pour récupérer le dialysat utilisé depuis le dispositif d'hémo-catharsis (2) vers la chambre de récupération ;
une pompe de dialysat (P2) disposée dans le passage de récupération de dialysat (14) ;
un passage d'alimentation en eau (15) pour fournir de l'eau purifiée à la chambre d'alimentation ;
une pompe d'alimentation en eau (P1) disposée dans le passage d'alimentation en eau (15) ;
un passage d'évacuation (16) pour évacuer le dialysat utilisé depuis la chambre de récupération ;
un passage d'élimination d'eau (27) pour connecter le passage de récupération de dialysat (14) et le passage d'évacuation (16) tout en contournant le récipient doseur (11, 12) ;
une pompe doseuse d'élimination d'eau (P5) disposée dans le passage d'élimination d'eau (27) ;
des moyens de commande (C) pour commander des opérations de la pompe doseuse d'élimination d'eau (P5), de la pompe d'alimentation en eau (P1) et de la pompe de dialysat (P2) ;
un passage de connexion (5) pour connecter un côté aval du passage d'alimentation en dialysat (13) et un côté amont du passage de récupération de dialysat (14) tout en contournant le dispositif d'hémo-catharsis (2) ;
un passage de circulation (28) pour connecter un côté amont du passage d'alimentation en eau (15) et un côté aval du passage d'évacuation (16), l'appareil étant configuré de telle sorte qu'un circuit fermé est formé par le passage de connexion (5) et le passage de circulation (28) au moment du nettoyage d'un circuit de dialysat (4) constitué des passages respectifs, et un liquide de nettoyage est distribué au circuit fermé pour nettoyer le circuit de dialysat ;
un réservoir de liquide de nettoyage (30) disposé pour fournir un liquide de nettoyage non dilué au circuit de dialysat ;
**caractérisé en ce que** :
l'appareil d'hémo-catharsis est configuré de sorte que, dans un état dans lequel la pompe doseuse d'élimination d'eau (P5) est arrêtée, les moyens de commande (C) sont configurés pour faire fonctionner la pompe d'alimentation en eau (P1) de sorte qu'une quantité prédéterminée du liquide de nettoyage non dilué est aspiré dans le circuit fermé à partir du réservoir de liquide de nettoyage (30), et sont en outre configurés pour faire fonctionner la pompe d'alimentation en eau (P1) et la pompe de dialysat (P2) pour faire circuler de l'eau purifiée et le liquide de nettoyage non dilué à travers le circuit fermé de telle sorte que le liquide de nettoyage est préparé en diluant le liquide de nettoyage non dilué avec de l'eau purifiée ; et
l'appareil d'hémo-catharsis comprend en outre une unité de détermination (D) configurée pour déterminer si une concentration du liquide de nettoyage dans le circuit fermé est appropriée et dans lequel l'appareil est configuré de sorte que, lorsqu'il est déterminé par l'unité de détermination (D) que la concentration du liquide de nettoyage dans le circuit fermé est appropriée, la pompe doseuse d'élimination d'eau (P5) est activée pour distribuer le liquide de nettoyage dans le passage d'élimination d'eau (27) et la pompe doseuse d'élimination d'eau (P5).

2. Appareil d'hémo-catharsis (1) selon la revendication 1, dans lequel l'unité de détermination (D) comprend une minuterie (T) qui décompte un temps pris pour faire circuler l'eau purifiée et le liquide de nettoyage non dilué, et l'unité de détermination (D) est configurée pour déterminer que la concentration du liquide de nettoyage dans le circuit fermé devient appropriée lorsque le temps de circulation décompté atteint un temps prédéterminé.

3. Appareil d'hémo-catharsis (1) selon la revendication 1, dans lequel l'unité de détermination (D) comprend un capteur de concentration (17), et l'unité de détermination est configurée pour déterminer que la concentration du liquide de nettoyage dans le circuit fermé est appropriée lorsqu'il est reconnu que le liquide de nettoyage non dilué circulant à travers le circuit fermé a été dilué pour avoir la concentration prédéterminée.

4. Appareil d'hémo-catharsis (1) selon l'une quelconque des revendications 1 à 3, comprenant en outre un passage de dialysat non dilué (21, 22) connecté au passage d'alimentation en eau (15) pour fournir un dialysat non dilué, et une pompe doseuse (P3, P4) pour le dialysat non dilué dans le passage de dialysat non dilué (21, 22), dans lequel l'appareil est configuré de telle sorte que lorsqu'il est déterminé par l'unité de détermination (D) que la concentration du liquide de nettoyage dans le circuit fermé est appropriée, la pompe doseuse pour le dialysat non dilué (P3, P4) est activée pour distribuer le liquide de nettoyage dans le passage de dialysat non dilué (21, 22) et la pompe doseuse pour le dialysat non dilué (P3, P4).

5. Appareil d'hémo-catharsis (1) selon la revendication 4, dans lequel :
chacune parmi la pompe doseuse d'élimination d'eau (P5) et la pompe doseuse pour le dialysat non dilué (P3, P4) comprend un cylindre cylindrique et un piston qui se déplace en va-et-vient à l'intérieur du cylindre ;
un passage de rinçage (38) est disposé pour fournir l'eau purifiée à une partie coulissante entre le piston et le cylindre ; et
l'appareil est configuré de telle sorte que, lorsqu'il est déterminé par l'unité de détermination (D) que la concentration du liquide de nettoyage dans le circuit fermé est appropriée, le liquide de nettoyage est distribué à travers le passage de rinçage (38).
